# EUROPEAN PATENT APPLICATION

(11) **EP 3 075 313 A1**
(43) Date of publication of application: **05.10.2016**
(21) Application number: 16157148.4
(22) Date of filing: 24.07.2014
(51) Int. Cl.: A61B 5/083, A61B 5/091, G06F 19/00

(54) **DETERMINING RESPIRATORY GAS EXCHANGE IN A SUBJECT**

(30) Priority: 25.07.2013 US 201361858178 P
(62) Divisional of application: 14829901.9
(71) Applicant: Meta Flow Ltd., 6200164 Tel-Aviv (IL)
(72) Inventor: MOR, Michal, 6200303 Tel-Aviv (IL); MOR, Merav, 6200303 Tel-Aviv (IL)
(74) Representative: Awapatent A/S

(57) **Abstract**

The present disclosure provides examples of a method, including: providing a representative inhale-exhale cycle breathing volume over time profile; and when the subject performs at least one inhale-exhale cycle that meets a correspondence criterion related to the representative profile, using data relating to oxygen consumption or carbon dioxide production during the inhale-exhale cycle that met the correspondence criterion to determine a metabolic property in the subject.

## Description

### TECHNICAL FIELD

The present disclosure is in the field of respiratory, oxygen consumption and carbon dioxide production analysis.

### SUMMARY

According to some embodiments, there is provided a method, comprising: providing a representative inhale-exhale cycle breathing volume over time profile; when the subject performs at least one inhale-exhale cycle that meets a correspondence criterion related to the representative profile, using data relating to oxygen consumption or carbon dioxide production during the inhale-exhale cycle that met the correspondence criterion to determine a metabolic property in the subject.

According to some embodiments, the method further comprises measuring oxygen consumption or carbon dioxide production while the subject is breathing, and when the subject performs at least one inhale-exhale cycle that meets the correspondence criterion, processing the oxygen consumption or the carbon dioxide production measurement taken during the at least one inhale-exhale cycle that met the correspondence criterion to determine the metabolic property of the subject.

According to some embodiments, the correspondence criterion is independent of the subject's oxygen consumption or carbon dioxide production.

According to some embodiments, the method further comprises presenting a current measured breathing volume over time of the subject relative to an inhale-exhale cycle breathing volume over time target profile.

According to some embodiments, the subject's representative profile is an *a priori* stored representative breathing volume over time of the subject during at least one inhale-exhale cycle.

According to some embodiments, the method further comprises: in a subject calibration phase: measuring a subject's breathing volume over time during a first plurality of inhale-exhale cycles; and when two or more inhale-exhale cycles from said first plurality inhale-exhale cycles meet a steady-state criterion, processing breathing volume over time from within the two or more inhale-exhale cycles, giving rise to the subject's representative breathing profile.

According to some embodiments, processing breathing volume over time during the two or more inhale-exhale cycles, comprises: computing a subject's representative inhale-exhale cycle breathing volume over time profile based on the subject's breathing volume over time during the two or more inhale-exhale cycles that met the steady-state criterion; obtaining an allowed deviation of breathing volume over time; and providing a target breathing profile based on the representative breathing profile and based on the allowed deviation.

According to some embodiments, the measurement phase includes measuring breathing volume over time during one or more inhale-exhale cycles until the correspondence criterion is met, and wherein the measurement phase is shorter than said calibration phase.

According to some embodiments, the measurement phase is at most five inhale-exhale cycles long.

According to some embodiments, during the measurement phase, in order to meet the correspondence criterion, the subject's current measured breathing volume over time during at least one inhale-exhale cycle needs to be within the allowed deviation throughout the at least one inhale-exhale cycle.

According to some embodiments, the representative profile is based on a breathing volume over time of a reference subject, and wherein the inhale-exhale cycle that is evaluated to determine compliance with the correspondence criterion is performed by a measured subject.

According to some embodiments, the method further comprises when the subject performs the at least one inhale-exhale cycle that meets the correspondence criterion, obtaining, during the at least one inhale-exhale cycle that met the correspondence criterion, an oxygen or carbon dioxide concentration measurements taken from the end of an exhalation which represents an alveolar volume.

According to some embodiments, the metabolic property is any one of a group consisting of: Rest Metabolic rate (RMR), Respiratory Energy Expenditure (REE), Respiratory Quotient (RQ) and Oxygen consumption.

According to some embodiments, there is provided an apparatus for determining oxygen consumption and carbon dioxide production in a subject, comprising:
(a) a storage module configured for storing a representative inhale-exhale cycle breathing volume over time profile;
(b) a processing unit configured to determine (i) when the subject performs at least one inhale-exhale cycle that meets a correspondence criterion related to the representative breathing profile, (ii) obtain data relating to oxygen consumption or carbon dioxide production during the inhale-exhale cycle that met the correspondence criterion, and (iii) determine, based on the data relating to oxygen consumption and carbon dioxide production, a metabolic property in the subject.

According to some embodiments, the apparatus further comprises an interface capable of presenting a target breathing profile using the data representative of the breathing profile, and the interface is configured to present a subject's current measured breathing volume over time relative to the target breathing profile.

According to some embodiments, the apparatus further comprises a sensor capable of measuring a subject's current breathing volume over time and oxygen consumption or carbon dioxide production, and wherein the sensor is configured to provide data representative of the subject's current breathing volume over time and oxygen consumption or carbon dioxide production.

According to some embodiments, there is provided a method, comprising: obtaining data related to a current event; obtaining a representative inhale-exhale cycle breathing volume over time profile during which cycle a subject's gas exchange represents a metabolic state of the subject; while a subject is under influence of the current event, when the subject performs at least one inhale-exhale cycle that meets a correspondence criterion related to the representative inhale-exhale cycle breathing volume over time profile, using data relating to oxygen consumption or carbon dioxide production during the inhale-exhale cycle that met the correspondence criterion to determine a metabolic effect of the event on the subject.

According to some embodiments, the method further comprises: obtaining reference metabolic data related to a first metabolic state of the subject; using data relating to oxygen consumption or carbon dioxide production during the inhale-exhale cycle that met the correspondence criterion to determine a second metabolic state of the subject, and wherein the metabolic effect of the event on the subject is derived from a relation between the first metabolic state of the subject and the second metabolic state of the subject.

According to some embodiments, there is provided an apparatus for determining a metabolic effect of an event on a subject, comprising: a storage module configured for storing data related to a current event; the storage module is configured for storing data related to a representative inhale-exhale cycle breathing volume over time profile during which cycle a subject's gas exchange represents a metabolic state of the subject; a processing unit configured to (i) determine when the subject performs at least one inhale-exhale cycle that meets a correspondence criterion related to the representative breathing profile, (ii) obtain data relating to oxygen consumption or carbon dioxide production during the inhale-exhale cycle that met the correspondence criterion, and (iii) determine, based on the data relating to oxygen consumption or carbon dioxide production and based on the stored data related to the current event, a metabolic effect of the event on the subject.

According to some embodiments, the apparatus further comprises an input interface allowing the user to specify an event that the user is currently under influence thereof.

According to some embodiments, there is provided a computer program product comprising a computer useable medium having computer readable program code embodied therein for determining oxygen consumption and carbon dioxide production in a subject, the computer program product comprising: computer readable program code for causing the computer to provide a representative inhale-exhale cycle breathing volume over time profile; computer readable program code for causing the computer to determine when the subject performs at least one inhale-exhale cycle that meets a correspondence criterion related to the representative breathing profile; computer readable program code for causing the computer to use data relating to oxygen consumption or carbon dioxide production during the inhale-exhale cycle that met the correspondence criterion to determine a metabolic property in the subject.

According to some embodiments, there is provided a computer program product comprising a computer useable medium having computer readable program code embodied therein for determining a metabolic effect of an event on a subject, the computer program product, comprising: computer readable program code for causing the computer to obtain data related to a current event; computer readable program code for causing the computer to obtain a representative inhale-exhale cycle breathing volume over time profile during which cycle a subject's gas exchange represents a metabolic state of the subject; computer readable program code for causing the computer to determine, while a subject is under influence of the current event, when the subject performs at least one inhale-exhale cycle that meets a correspondence criterion related to the representative inhale-exhale cycle breathing volume over time profile; and computer readable program code for causing the computer to use data relating to oxygen consumption or carbon dioxide production during the inhale-exhale cycle that met the correspondence criterion to determine a metabolic effect of the event on the subject.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
FIG. 1 is a block diagram illustration of an apparatus for determining oxygen consumption or carbon dioxide production in a subject, according to examples of the presently disclosed subject matter;
FIG. 2 is a flowchart illustration of a method of determining oxygen consumption or carbon dioxide production in a subject, according to examples of the presently disclosed subject matter;
FIG. 3 is a graphical illustration of a subject's inhalation and exhalation volume vs. time as measured during a process of determining the subject's representative inhale-exhale cycle breathing volume over time profile, as part of examples of the presently disclosed subject matter;
FIG. 4 is a graphical illustration of breathing parameters which can be used to characterize a breath, according to examples of the presently disclosed subject matter;
FIG. 5 is a graph illustrating one possible representation of a target breathing profile, according to examples of the presently disclosed subject matter;
FIG.6 is graph that illustrates an inhale-exhale cycle performed by the subject which meets the correspondence criterion that is also shown as a graph with margins that indicate an allowed deviation, according to examples of the presently disclosed subject matter;
FIG. 7 is graph that illustrates an inhale-exhale cycle performed by the subject which does not meet the correspondence criterion, according to examples of the presently disclosed subject matter;
FIG. 8 is a graphical illustration of a representation of a current measured breathing volume over time of a subject relative to the representative breathing profile, which can be displayed to the user in real-time, according to examples of the presently disclosed subject matter.
FIG. 9 is a graphical illustration a set of stored gas exchange measurements that were obtained as part of the method of determining a metabolic effect of an event on a subject. Each row represents a different measurement;
FIG. 10 is a block diagram illustration of an apparatus for determining an effect of an event over metabolic properties of a subject, according to examples of the presently disclosed subject matter;
FIG. 11 is a flowchart illustration of a method of determining an effect of an event over metabolic properties of a subject, according to examples of the presently disclosed subject matter;
FIG. 12 is a graphical illustration of a data structure in which various data related to recorded events can be kept, as part of some examples of the presently disclosed subject matter; and
FIG. 13 is a graphical illustration of a data structure in which various data related to different subjects can be kept, as part of examples of the presently disclosed subject matter.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION

In the following detailed description, numerous specific details are set forth in order to provide a thorough understanding of the presently disclosed subject matter. However, it will be understood by those skilled in the art that the presently disclosed subject matter may be practiced without some of these specific details. In other instances, well-known methods, procedures and components have not been described in detail so as not to obscure the presently disclosed subject matter.

Unless specifically stated otherwise, as apparent from the following discussions, it is appreciated that throughout the specification various functional terms refer to the action and/or processes of a computer or computing device, or similar electronic computing device, that manipulate and/or transform data represented as physical, such as electronic, quantities within the computing device's registers and/or memories into other data similarly represented as physical quantities within the computing device's memories, registers or other such tangible information storage, memory, transmission or display devices.

It is appreciated that, unless specifically stated otherwise, certain features of the presently disclosed subject matter, which are, for clarity, described in the context of separate embodiments, may also be provided in combination in a single embodiment. Conversely, various features of the presently disclosed subject matter, which are, for brevity, described in the context of a single embodiment, may also be provided separately or in any suitable subcombination.

As used herein, the terms "example", "for example," "such as", "for instance" and variants thereof describe non-limiting embodiments of the presently disclosed subject matter. Reference in the specification to "one case", "some cases", "other cases" or variants thereof means that a particular feature, structure or characteristic described in connection with the embodiment(s) is included in at least one embodiment of the presently disclosed subject matter. Thus the appearance of the phrase "one case", "some cases", "other cases" or variants thereof does not necessarily refer to the same embodiment(s).

The operations in accordance with the teachings herein may be performed by a computer specially constructed for the desired purposes or by a general purpose computer specially configured for the desired purpose by a computer program stored in a tangible computer readable storage medium.

Many of the functional components of the presently disclosed subject matter can be implemented in various forms, for example, as hardware circuits comprising custom VLSI circuits or gate arrays, or the like, as programmable hardware devices such as FPGAs or the like, or as a software program code stored on an tangible computer readable medium and executable by various processors, and any combination thereof. A specific component of the presently disclosed subject matter can be formed by one particular segment of software code, or by a plurality of segments, which can be joined together and collectively act or behave according to the presently disclosed limitations attributed to the respective component. For example, the component can be distributed over several code segments such as objects, procedures, and functions, and can originate from several programs or program files which operate in conjunction to provide the presently disclosed component.

In a similar manner, a presently disclosed component(s) can be embodied in operational data or operational data can be used by a presently disclosed component(s). By way of example, such operational data can be stored on a tangible computer readable medium. The operational data can be a single data set, or it can be an aggregation of data stored at different locations, on different network nodes or on different storage devices. Embodiments of the presently disclosed subject matter are not described with reference to any particular programming language. It will be appreciated that a variety of programming languages may be used to implement the teachings of the presently disclosed subject matter as described herein.

Unless specifically stated otherwise, as apparent from the following discussions, it is appreciated that throughout the specification discussions utilizing terms such as "processing", "calculating", "measuring", "using", "determining", "generating", "setting", "configuring", "selecting", "searching", "storing", or the like, include actions and/or processes of a computer that manipulate and/or transform data into other data, said data represented as physical quantities, e.g., such as electronic quantities, and/or said data representing the physical objects. The terms "computer", "processor", and "controller" should be expansively construed to cover any kind of electronic device with data processing capabilities.

According to an aspect of the presently disclosed subject matter, there is provided a method of determining a metabolic property in a subject. According to examples of the presently disclosed subject matter, the method can include: providing a representative inhale-exhale cycle breathing volume over time profile; and when the subject performs at least one inhale-exhale cycle that meets a correspondence criterion related to the representative profile, using data relating to oxygen consumption or carbon dioxide production during the inhale-exhale cycle that met the correspondence criterion to determine a metabolic property of the subject.

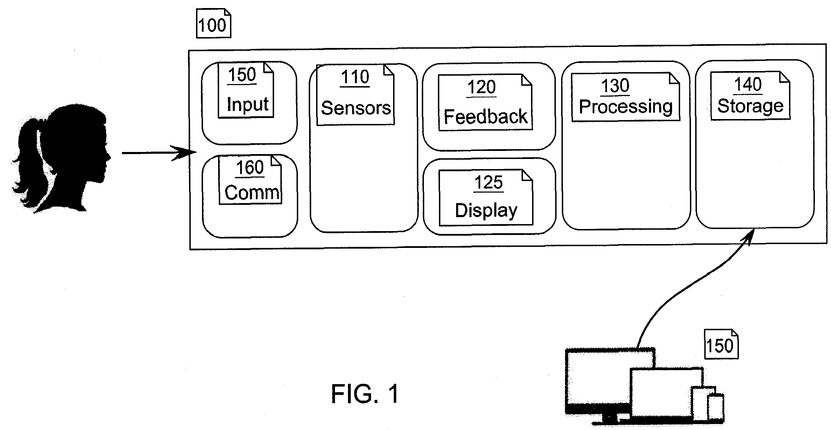

Reference is now made to **FIG. 1****,** which is a block diagram illustration of an apparatus for determining a metabolic property in a subject, according to examples of the presently disclosed subject matter. According to examples of the presently disclosed subject matter, the apparatus 100 for determining a metabolic property in a subject can include one or more sensor 10, a storage module 40 and a processing unit 30. The apparatus can further include an input interface 50 and an output interface 20. The apparatus can also include a communication module 60. By way of example, the apparatus can be a smart phone, a computer or a dedicated computerized device that is using generic and/or application specific hardware, possibly in combination with software. In would be appreciated that the device 100 can be implemented in many other forms, including as a distributed device which is comprised of several interconnected nodes. For example, there can be provided a sensing device, which can include one or more sensors a communication module and possibly also a processing unit and a storage unit. The sensing device can be configured to measure gas exchange in a subject's breath and possibly preform some processing of the measured data. The sensing device can communicated the measured data to a smartphone device, over a wired or a wireless channel, and the smartphone device can run a software program which further processes the measurements provided by the sensing device and present feedback and other data to a user. In a further example, the sensors, and the processing performed by the sensing device can be incorporated into the smartphone, and the entire process can run on the smartphone device. In yet another example, whether the sensing device is part of the smartphone or not, a cloud based platform 150 can be coupled (typically via wireless communication) to the smartphone device, and the measurements provided by the sensor or some derivative thereof, can be uploaded to the cloud platform 150. On the cloud platform 150, the measurements (or the derivatives) can be further processed, and the processed data can be sent back to the smartphone device where further processing can take place, or where the cloud generated data is presented to the user. The cloud generated data can also be presented to other devices. In this regard, it would be appreciated that the apparatus shown in FIG. 1 and described herein particularly with reference to FIG. 1, is a mere example of one possible implementation of a device for determining a metabolic property in a subject according to the presently disclosed subject matter.

According to examples of the presently disclosed subject matter, the storage module 40 can store a representative breathing profile embodied in digital data. According to examples of the presently disclosed subject matter, the representative breathing profile is an inhale-exhale cycle breathing volume over time profile. Thus, for example, the representative breathing profile can be an *a priori* stored representative breathing volume over time of a subject during at least one inhale-exhale cycle. Still further by way of example, for each subject or user a representative breathing profile is provided.

According to one example, the representative breathing profile can include a set of values which correspond to breathing volume over time during at least one inhale-exhale cycle. Still further by way of example, the data representing the representative breathing profile can include a set of values which correspond to a representative inhale-exhale cycle breathing volume over time profile at a plurality of selected inhale-exhale cycles. Still further by way of example, the representative breathing profile is a subject's gas exchange cycle (breath) which represents a metabolic state of the subject. Examples of methods that can be used for selecting inhale-exhale cycles which can be used for providing the representative breathing profile are described separately as part of the presently disclosed subject matter and include (but are not limited to) various methods that use the metabolic properties extraction "gold standard". Examples of known methods that use metabolic properties extraction "gold standard".

Reeves, Marina M., et al. "Reducing the time period of steady state does not affect the accuracy of energy expenditure measurements by indirect calorimetry." Journal of Applied Physiology 97.1 (2004): 130-134. describe how to calculate steady state breathing and extracting metabolic properties according to "gold standard" and can be used in examples of the presently disclosed subject matter to obtain the representative breathing profile.

The representative breathing profile data can also include a set of values which correspond to a deviation of breathing volume over time from the representative inhale-exhale cycle breathing volume over time profile. The deviation can be used to allow some tolerance during the measurement phase when the representative profile is used to determine a metabolic property in a subject. An example of the manner by which the representative breathing profile can be generated is provided below.

According to examples of the presently disclosed subject matter, the output interface 20 can be configured to present a target breathing profile using the data representative of the inhale-exhale cycle breathing volume over time profile. For example, the target breathing profile can be a visual display of a current volume of time of a subject's breathing relative to and in synchronization with a visual display which corresponds to the representative inhale-exhale cycle breathing volume over time profile. According to examples of the presently disclosed subject matter, the output interface 20 can be a digital display unit, such as an LCD display, a touch-screen, an OLED display, etc. In further examples, in other any type of device that is capable of presenting to a subject the target breathing profile can be used, including devices that operate by providing acoustic indication (sound), such as speakers, sensory devices, etc. By way of example, the output interface 20 can include a speaker which generates sound and the apparatus 100 can include a further output interface 25 such as display for presenting graphs. The output interface can include multiple windows, tabs or any other distinct display area (or representation of any other sort), in which details regarding the current metabolic properties and other feedback or information can be displayed. The metabolic properties can include current metabolic properties and possibly historical metabolic data as well. For example, the output interface 25 can provide a visual representation of the metabolic property of the subject.

According to examples of the presently disclosed subject matter, the representative breathing profile, the target breathing profile and the metabolic property can be generated based on the breathing properties of the same person (or the same subject).

In other examples of the presently disclosed subject matter, the representative breathing profile is a representative breathing volume over time of a reference subject, and the inhale-exhale cycle which is measured to determine a current breathing volume over time (for determining the metabolic property in a subject) is performed by a measured subject. The reference subject and the measured subject may not be the same person. In a further example, the reference subject is not necessarily associated with a real person or with a particular person. For example, the representative breathing profile and the target breathing profile can be simulated or can be generated by measuring respiratory properties of a different person than the person whose breathing is used to determine a metabolic property of the person, or in another example the representative breathing profile and the target breathing profile can be generated by measuring respiratory properties of a group of persons. In cases where the representative breathing profile and the target breathing profile is associated with different person(s) than the person whose breathing is used to determine a metabolic property in the person, there can be some correlation between the reference subject(s) and the measured subject.

Various metabolic properties are known in the art. Examples of metabolic properties as this term is used herein include a Rest Metabolic rate (RMR), Respiratory Energy Expenditure (REE), Respiratory Quotient (RQ) and Oxygen consumption.

The processing unit 30 can be configured to determine when at least one inhale-exhale cycle meets a correspondence criterion related to the breathing profile, as will be further described below. The processing unit 30 can also be configured to obtain data relating to oxygen consumption or carbon dioxide production (or both) during the inhale-exhale cycle that met the correspondence criterion, and to determine a metabolic property in the subject based on the data relating to oxygen consumption or carbon dioxide production, as will also be further described below.

As mentioned above, the representative breathing profile data is stored in the storage unit 40. According to examples of the presently disclosed subject matter, the representative breathing profile data can be provided as input from an external source that is operatively connected to the apparatus 100. Examples of possible external sources can include a sensor or a sensing device that is capable of measuring breathing volume over time during at least one inhale-exhale cycle of a subject; a remote computer in which the data representing the breathing profile was stored; and data provided through an input interface 50, e.g., as manual input by a user of the apparatus.

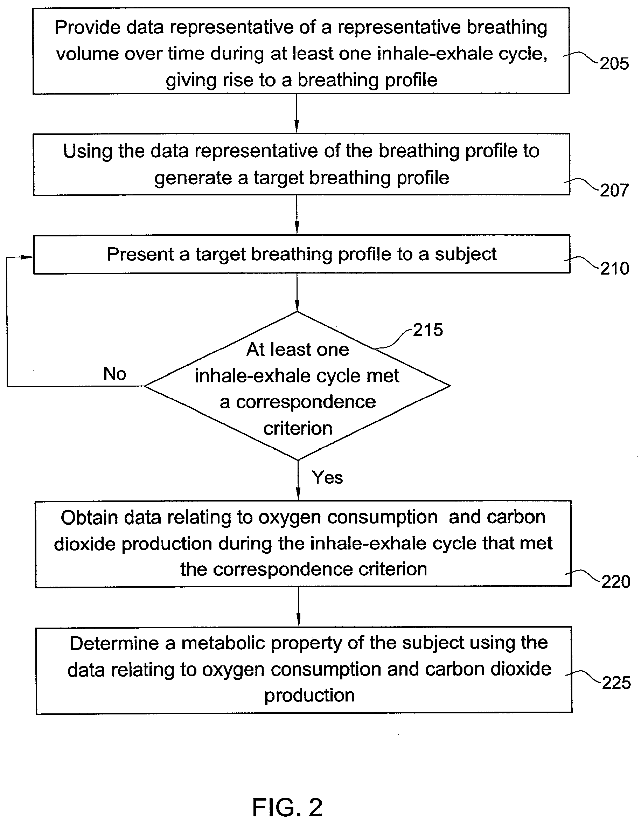

Reference is now additionally made to **FIG. 2****,** which is a flowchart illustration of a method of determining oxygen consumption or carbon dioxide production in a subject, according to examples of the presently disclosed subject matter. For convenience, the description of the method illustrated in **FIG. 2** is made with reference to the apparatus 100 shown in FIG. 1 and the various components of the apparatus 100. It would be appreciated however, that in some examples of the presently disclosed subject matter, the method of determining oxygen consumption or carbon dioxide production in a subject is not necessarily bound to be implemented in apparatus 100, and rather any other suitable device or system can be used to implement the various examples of the of determining oxygen consumption or carbon dioxide production in a subject which are described herein.

According to examples of the presently disclosed subject matter, a breathing volume over time during at least one inhale-exhale cycle data which is to be used as a representative breathing profile can be provided (block 205). As mentioned above, the representative breathing profile can be stored in the storage unit 40.

There is now provided a description of a process protocol which can be used to generate a representative breathing profile. It should be noted that this protocol is provided here as an example, and that other protocols and other ways can be devised to generate a representative breathing profile of a subject.

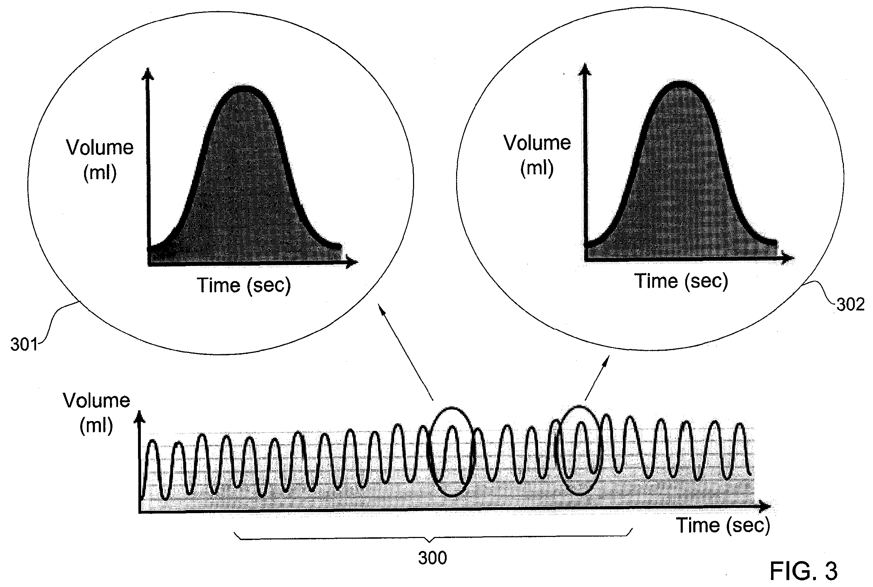

According to examples of the presently disclosed subject matter, the representative breathing profile can be generated by recording a subject's breathing when the subject's breathing is in a steady state. Further by way of example, the subject's breathing can be monitored for about 3-10 minutes. Typically the subject's inhalation and exhalation volume vs. time is measured. **FIG. 3** is a graphical illustration of a subject's inhalation and exhalation volume vs. time as measured during a process of determining the subject's steady state breathing (chart 300), as part of examples of the presently disclosed subject matter. Graph 301 and graph 302 are enlarged views of the two inhale-exhale cycles which were performed during the process of determining the subject's steady state breathing which met a steady state criterion. An example of a steady state criterion is disclosed in Reeves, et al. Still further by way of example, the analysis on each breath (a discrete inhale-exhale cycle) can include, for example, calculations of: Vin, Vout, Tin, Tout, Ttotal, Frequency and Standard Deviation of each of these measures, where Vin denotes the volume of air during the inhalation, Vout denote the volume of air during the exhalation, Tin: denotes the time of breath during the inhalation (the duration of the inhalation), Tout denotes the time of breath during the exhalation (the duration of the exhalation), Ttotal denotes the total time of breath (the breath duration), and Frequency is the number of breathes per minute.

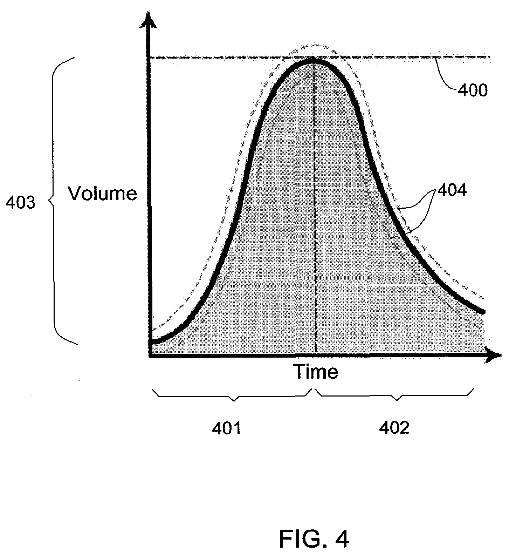

**FIG. 4** is a graphical illustration of breathing parameters which can be used to characterize a breath, as part of examples of the presently disclosed subject matter. In particular, the breathing profile shown in FIG. 4 can provide an illustration of how a representative inhale-exhale cycle breathing volume over time profile.

In the inhale-exhale cycle breathing volume over time profile shown in FIG. 4, the Y-axis represents the volume, in particular the inhale volume Vin 403 of a subject's breath, the X-axis represents time or in this case Ttotal 400 or the duration of the breath (inhale exhale cycle), where segment 401 represents Tin which is the inhale period, and Tout 402 represents the exhale period. SD line 404 represents a tolerance (e.g., standard deviation) profile or in this case a standard deviation which is acceptable according to the correspondence criterion that is used to determine a metabolic property in a subject. Using these parameters, an analysis can be performed and one or more (e.g., one, two, ..., n) breaths, i.e., one or more inhale-exhale cycles, can be selected for providing the representative breathing profile of the subject.

It would be appreciated that for example, in case SD of Vin and Ttotal or SD of oxygen consumption (see Hill, RICHARD W. "Determination of oxygen consumption by use of the paramagnetic oxygen analyzer." J. appl. Physiol 33.2 (1972): 261-263 for an example of a calculation of SD of oxygen consumption) are used to identify breaths which represent a steady state breathing profile, two or more (e.g., 2, 3, ..., n) inhale-exhale cycles are selected to be representative of the steady state breathing profile of the subject. For example, between 3-10 breaths are typically selected to be representative of the breathing profile.

Further by way of example, two or more inhale-exhale cycles are selected to be representative of the steady state breathing profile of the subject when a steady state condition is met. Still further by way of example, the steady state condition can be associated with SD of Vin vs. time and SD of Ttotal vs. time or SD of oxygen consumption vs. time in a subject's breaths. Yet further by way of example, the steady state condition can require that the SD of Vin vs. time and the SD of Ttoatl vs. time or SD of oxygen consumption vs. time in a subject's breaths be below (or above) certain thresholds or within a certain range. Still further by way of example, the SD of Vin vs. time and the SD of Ttoatl vs. time or SD of oxygen consumption vs. time thresholds can be predefined, or in a further example, the SD of Vin vs. time and the SD of Ttoatl vs. time or SD of oxygen consumption vs. time thresholds can be determined or adapted for each subject (e.g., according to subject height, gender, weight and/or any other personal characteristic of the subject) or depending on other physiological factors.

As can be seen in FIG. 3, by way of example, several breaths can be selected to be representative of the breathing profile (graphs 302 and 303), according to the steady state condition, which is associated with SD of Vin vs. time and the SD of Ttoatl vs. time or SD of oxygen consumption vs. time in a subject's breaths, and a statistical processing can be applied over the selected breaths to provide the representative breathing profile. Still further by way of example, using the statistical processing over the several breaths which are selected for calculating the representative breathing profile, the representative breathing profile can be provided as data representative of a breathing volume over time during a single inhale-exhale cycle with some allowed deviation. It would be noted that many different processing steps can be used to calculate the breathing volume over time and the allowed deviation in the breathing profile, and that for any given set of several breaths which are selected to be representative of the breathing profile, different breathing profiles can be generated, depending on implementation of the statistical processing operation.

The breathing profile of the subject can be based on and takes into account additional or alternative statistical and other measures such as: subject's gender, subject's age, subject's weight, subject's height. For example, such measure can be used to factor some pre-existing representative breathing profile.

Resuming now the description of **FIG. 2****,** according to examples of the presently disclosed subject matter, a target breathing profile can be presented to a subject (block 210). According to examples of the presently disclosed subject matter, the target breathing profile can be created using the data representative of the steady-state breathing profile (block 207).

For example, the target breathing profile can include a set of values which correspond to breathing volume over time during at least one inhale-exhale cycle. Still further by way of example, the target breathing profile can include a set of values which correspond to a representative subject's breathing volume over time during an inhale-exhale cycle, and a set of values which correspond to an allowed deviation from the representative subject's breathing volume over time. Still further by way of example, the correspondence criterion of a breathing profile can be an allowed deviation of the tidal volume and the time of breath from the average of the steady state breathing the average of the steady state breathing. This can be represented as a set of values or ranges, thresholds, graphs, etc. Other examples of correspondence criterion can be associated with averaging values and as long as the deviation of the average from a representative breathing profile (as defined below) is less than a threshold, the correspondence criterion would be fulfilled. In another example, any measured sample must be within a certain allowed deviation from a representative breathing profile, and in case one or more samples are outside the allowed deviation, the correspondence criterion fails.

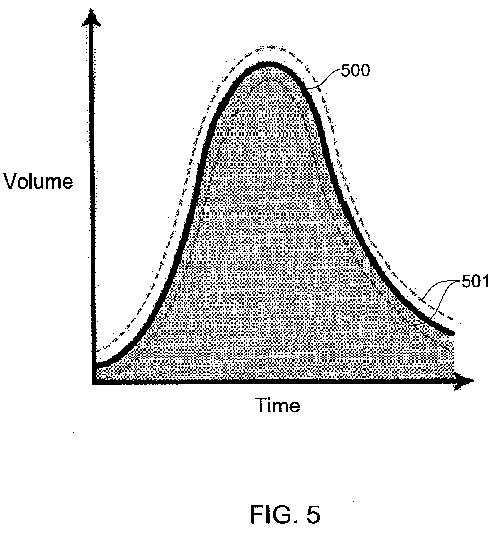

According to examples of the presently disclosed subject matter, the target breathing profile can be represented or can be provided as a graph or a set of graphs, such as the graph shown by way of example in **FIG. 5** where line 501 is the target subject's breathing volume over time during an inhale-exhale cycle and lines 502 illustrate the allowed deviation. According to one example, the target breathing profile 501 and the allowed deviation 502 provide the representative breathing profile. Further by way of example, the target breathing profile 501 and the allowed deviation 502 represent a breathing cycles that can be used to determine a metabolic state of the subject.

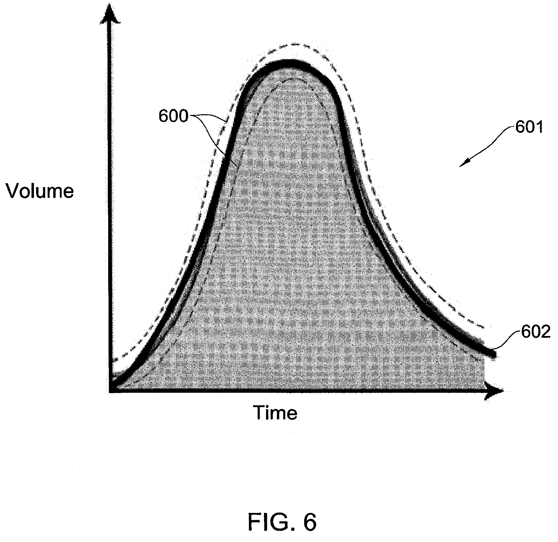

According to examples of the presently disclosed subject matter, the subject may perform one or more inhale-exhale cycles, and when the subject performs at least one inhale-exhale cycle that meets a correspondence criterion (block 215) related to the representative breathing profile, data relating to oxygen consumption and carbon dioxide production during the inhale-exhale cycle that met the correspondence criterion can be obtained (block 220). The obtained oxygen consumption and carbon dioxide production data can be used to determine a metabolic property in the subject (block 225). **FIG. 6** is graph that illustrates an inhale-exhale cycle performed by the subject juxtaposed over a representative breathing profile. In the scenario shown in FIG. 6, the correspondence criterion that is used to determine when to extract data relating to oxygen consumption or carbon dioxide production from a breath performed by the subject and use it to determine a metabolic property in the subject is associated with the target breathing profile 501 shown in FIG. 5, and the margins 502 which represent an allowed deviation.

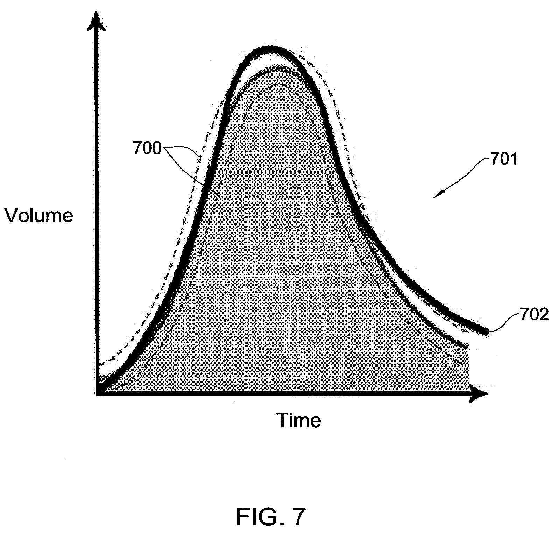

As can be seen in FIG. 6, the measured inhale-exhale cycle performed by the subject 601 shown in FIG. 6, meets the correspondence criterion that is also shown as a graph with margins that indicate allowed deviation. According to examples of the presently disclosed subject matter, as long as the subject does not meet the correspondence criterion (e.g., see the state at **FIG. 7****),** presentation of the target breathing profile (block 210) can be resumed, e.g., at one or more subsequent breaths.

**FIG. 7** is graph that illustrates an inhale-exhale cycle performed by the subject juxtaposed over a representative breathing profile, but unlike the scenario shown in FIG. 6, in FIG. 7 the breath 701 performed by the subject is outside the margins 502 around the target breathing profile 501 and so does not meet the correspondence criterion. It should be noted that the correspondence criterion in some implementation can allow some (very short and/or very small) deviations from the target breathing profile (the margins 502).

According to examples of the presently disclosed subject matter, the sensor 10, which is used by or with the device, can be capable of measuring oxygen and/or carbon dioxide concentration in the subject's breath. When it is determined that a certain breath (an inhale-exhale cycle) meets the correspondence criterion, data relating to the oxygen and/or carbon dioxide concentration in the subject's breath (the breath which met the criterion) is obtained from the sensor 10. According to examples of the presently disclosed subject matter, the sensor 10 can include a chamber and can be capable of capturing the end exhalation volume of a breath. When it is determined that a certain breath meets the correspondence criterion, the end exhalation volume captured in the sensor is sensed to determine oxygen and/or carbon dioxide concentration. It would be appreciated that other sensing techniques, and other types of sensors can be used to measure the oxygen concentration and/or carbon dioxide production, in accordance with further examples of the presently disclosed subject matter. One example of a sensor which may be used to measure oxygen concentration in a subject's breath is an electro-chemical oxygen sensor, such as oxygen sensor catalog number OOM103-1M retailed by EnviteC-Wismar GmbH, a Honeywell Company residing in Wismar, Germany. One example of a sensor which may be used to measure carbon dioxide production in a subject's breath is an optical carbon dioxide sensor, such as carbon dioxide sensor catalog number CO2F-W retailed by SST. It should be noted that two or more (three, four, etc.) can be used in combination to provide the measurements.

According to examples of the presently disclosed subject matter, metabolic properties of the subject can be calculated, e.g., by the processor 30, using the data related to the breath that met the correspondence criterion, which is obtained from the sensor 10. According to an example, the metabolic properties of the subject can be calculated using a respiratory volume pattern and an oxygen consumption and carbon dioxide production calculation based on the oxygen and/or carbon dioxide concentration measurement, Reference to this calculation can be found in the following scientific literature: see for example Weir, JB de V. "New methods for calculating metabolic rate with special reference to protein metabolism." The Journal of physiology 109.1-2 (1949): 1.

According to examples of the presently disclosed subject matter, a subject's oxygen consumption and/or carbon dioxide production calculation can be based at least on data that is obtained from three sensors, a flow meter sensor and an oxygen concentration sensor and a carbon dioxide concentration sensor. Other sensors also can be used. Further by way of example, oxygen consumption and carbon dioxide production can be calculated using a volume vs. time output from the flow meter, with the oxygen and carbon dioxide concentration that is provided as output by the oxygen and carbon dioxide sensors, respectively. Still further by way of example, volume vs. time can be continuously measured by the flow meter which monitors the subject's breath. Oxygen and carbon dioxide concentration measurement can be performed at least once per-breath, using the oxygen and carbon dioxide sensors. In accordance with one example of the presently disclosed subject matter, oxygen and carbon dioxide concentration measurement can be performed at most once per-breath, on the end exhalation air of the subject's breath, using the oxygen and carbon dioxide sensors.

According to examples of the presently disclosed subject matter, the processing unit 30 can be configured to store and process some of the data from the sensor, e.g., the oxygen and/or carbon dioxide concentration data, only when it is determined that the breath with which the data is associated met the correspondence criterion. Otherwise, the data can be discarded.

Using oxygen and/or carbon dioxide concentration, the oxygen consumption and/or carbon dioxide production can be calculated. It should be appreciated that the term sensor 10 is used herein in a broad sense, and the sensor can include one, two or more (e.g., n) different sensors which operate together to measure breath related properties, including: volume vs. time, oxygen and/or carbon dioxide concentration, etc.

By way of example, oxygen consumption can be calculated as the inhalation volume multiple of the oxygen concentration in the inhale air minus the dead space volume multiple of the oxygen concentration in the inhaled air and minus the difference between the exhalation volume to the dead space volume multiple of the oxygen concentration in the exhale. Still further by way of example, the physiological dead space can be calculated based on weight, age, gender, height and other personal characteristics of the subject.

Yet further by way of example, the oxygen consumption calculation can be assuming Ambient Temperature and Pressure Saturated units (ATPS), Further calculation can be carried out to convert to the ATPS figures to Standard Temperature and Pressure Dry units (STPD) and then to Kcal. It would be noted that the computation can be adapted if necessary for different pressure, temperature and other ambient conditions as necessary.

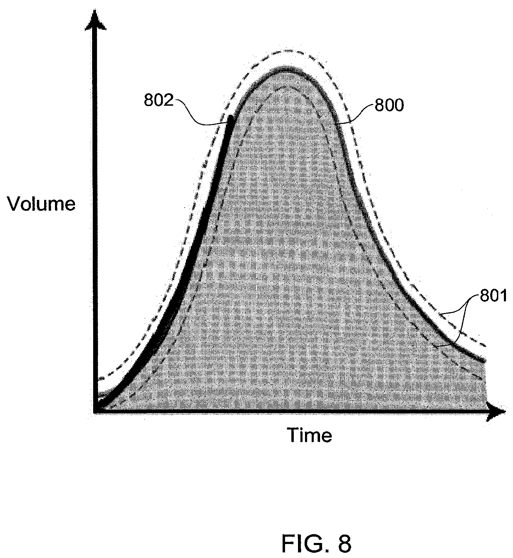

According to examples of the presently disclosed subject matter, the presentation of a current measured breathing volume over time of a subject relative to the representative breathing profile can be generated and rendered in real-time so as to allow the subject instant feedback. Reference is made to **FIG. 8****,** which is a graphical illustration of a representation of a current measured breathing volume over time of a subject relative to the representative breathing profile, which can be displayed to the user in real-time, according to examples of the presently disclosed subject matter. As can be seen in **FIG. 8****,** a subject's current breathing volume vs. time 801 can be shown relative to an allowed breathing volume vs. time range, which is a representation of the representative breathing profile. As mentioned above, the representative breathing profile can include a set of values 501 which correspond to representative subject's breathing volume over time during the one or more inhale-exhale cycles, and a set of values which correspond to an allowed deviation 502 of breathing volume over time from the representative subject's breathing volume over time.

By way of non-limiting example, the typical duration of a calibration phase during which the representative breathing profile of a subject is determined, and which typically requires the subject's breathing to naturally reach its steady state, is approximately 10-15 minutes long. Still further by way of non-limiting example, an average subject can successfully mimic, within an acceptable deviation, the representative breathing profile, when presented with instant feedback, as suggested herein, within a period of 1-2 minutes.

Having described an aspect of the presently disclosed subject matter which can be used, by way of example, to shorten the duration and possibly also the complexity of metabolic property measurement in a subject, there is now provided a description of a further aspect of the presently disclosed subject matter, which relates to a method and apparatus determining a metabolic effect of an event on a user, to program a storage device readable by machine, tangibly embodying a program of instructions executable by the machine to perform a method of determining a metabolic effect of an event on a subject, and to a computer program product comprising a computer useable medium having computer readable program code embodied therein for determining a metabolic effect of an event on a subject.

It would be appreciated, that in some cases, an effect of an event over metabolic properties of a subject can be relatively short, or the frequency by which the subject is interested to determining the metabolic effect of different (or same) events can be too high for time-consuming metabolic measurement technologies of the prior art. Accordingly in some cases, the method and apparatus for determining a metabolic effect of an event on a user, the program storage device readable by machine, tangibly embodying a program of instructions executable by the machine to perform a method of determining a metabolic effect of an event on a subject, and the computer program product comprising a computer useable medium having computer readable program code embodied therein for determining a metabolic effect of an event on a subject, the computer program product, which are described herein can be implemented in accordance with the teachings provided above.

Thus, according to an aspect of the presently disclosed subject matter, a method determining a metabolic effect of an event on a subject can include: obtaining data related to a current event; obtaining a representative inhale-exhale cycle breathing volume over time profile during which cycle a subject's gas exchange represents a metabolic state of the subject; while a subject is under influence of the current event, when the subject performs at least one inhale-exhale cycle that meets a correspondence criterion related to the representative inhale-exhale cycle breathing volume over time profile, using data relating to oxygen consumption or carbon dioxide production during the inhale-exhale cycle that met the correspondence criterion

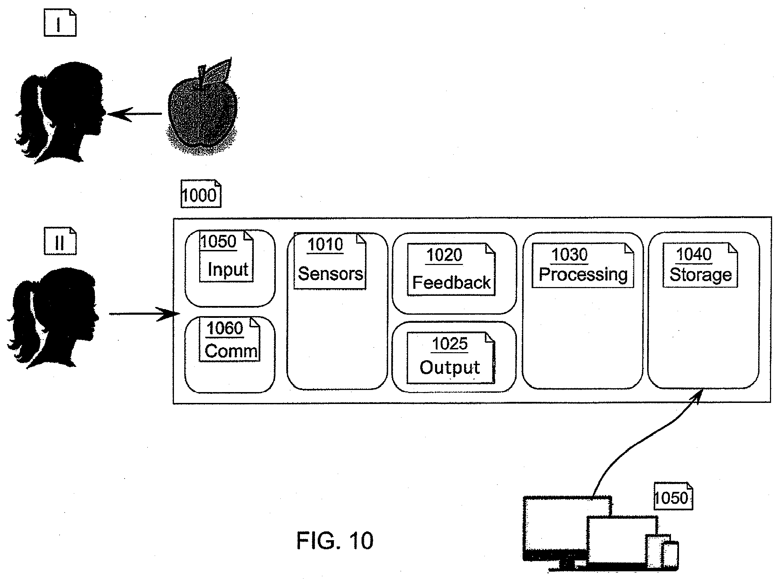

Reference is now made to **FIG. 10****,** which is a block diagram illustration of an apparatus for determining an effect of an event over metabolic properties of a subject, according to examples of the presently disclosed subject matter. The apparatus 1000 in FIG. 10 is similar in design and includes similar components to apparatus 100, which was described herein above. According to examples of the presently disclosed subject matter, apparatus 1000 can have similar capabilities as apparatus 100, described above. Furthermore, apparatus 1000 can include a storage module 1010 and a processing unit 1030 which are capable of operating in a manner which is similar to the operation of the storage module 40 and the processing unit 30 described above. According to examples of the presently disclosed subject matter, an output interface 1020 of apparatus 1000 can be configured to provide the subject an output screen (or output in any other suitable form) which is similar to the output screen shown in FIG. 1 and described above in detail. The input interface 1040 can have similar capabilities as the input interface 50.

According to examples of the presently disclosed subject matter, the apparatus 1000 and its various components can be capable of further operations, as will now be disclosed. According to examples of the presently disclosed subject matter, the input interface 1040 can be capable of receiving data from a user regarding a current event. For example, the user can be the subject which is under the influence of the current event, and the data from a user regarding a current event can be provided through the input interface 1040. By way of example, the data related to the current event can be a name of the event or an identifier of the type of the event (an event type classifier code) or an image of an object with which the event is associated. Still further by way of example, the data related to the event can provide a unique identifier of the event type. For example, the event can be "subject ate an apple", and this event type can be associated with a certain unique identification code. The subject can provide the identifier of "subject ate an apple" which can be used to characterize further operations of the apparatus 1000, as will be disclosed herein.

According to examples of the presently disclosed subject matter, the output interface 1020 can be capable of operating in a manner which is similar to the operation of output interface 20, including the representation of the target breathing profile, including the allowed deviation, and the real-time representation of a subject's inhalation and exhalation volume vs. time. According to examples of the presently disclosed subject matter, the apparatus 1000 can include a further output interface 1025, or can display another window, tab or any other distinct display area (or representation of any other sort), in which details regarding the current event and regarding various metabolic properties can be displayed. The metabolic properties can include current metabolic properties and possibly historical metabolic data as well. For example, the output interface 1025 can provide a visual representation of the effect of the event over the metabolic property of the subject.

It would be appreciated that additional data can be provided with the data relating to the event, including for example, an ID of the subject, data regarding factors, in particular current temporary state or factors, which may influence the metabolism of the subject, etc.

According to examples of the presently disclosed subject matter, at least part of the data related to the current event can be prestored in the storage unit 1010, and for example, the user can select from prestored data a subset of the data which is associated with the current event. Further by way of example, the data related to the current event can include a description of the event, a classification of the event, and historical metabolic data of the user which is related to the event.

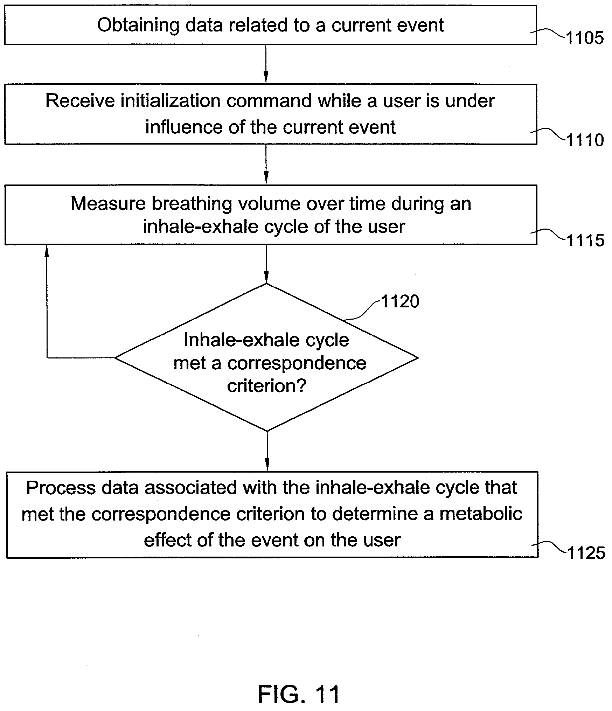

Reference is now additionally made to **FIG. 11****,** which is a flowchart illustration of a method of determining an effect of an event over metabolic properties of a subject, according to examples of the presently disclosed subject matter. Initially, at block 1105, the data related to the current event can be obtained. While the subject is under the influence of the event, the determination of the effect of the event over metabolic properties can be initiated (block 1110). The initialization of the determination can be explicit or can be triggered by receipt of the data related to the current event. According to examples of the presently disclosed subject matter, while the subject is under influence of the current event, the subject's breathing volume over time during an inhale-exhale cycle can be measured (block 1115). The subject's measured breathing volume over time during an inhale-exhale cycle can then be evaluated, to determine whether it meets a correspondence criterion (block 1120). The measurement of the breathing volume over time during an inhale-exhale cycle can be performed according to the examples described above. The analysis of the breathing volume over time can also be performed according to the examples described above.

According to examples of the presently disclosed subject matter, when the subject performs at least one inhale-exhale cycle that meets a correspondence criterion related to a steady-state breathing profile, data relating to oxygen consumption or carbon dioxide production during the inhale-exhale cycle that met the correspondence criterion can be used to determine a metabolic effect of the current event on the subject (block 1125). Otherwise, block 1115 can be repeated one or more additional times (e.g., two, three, ..., n times) at least until the correspondence criterion is met or until the process is terminated. It would be noted that the representative breathing inhale-exhale cycle breathing volume over time profile which was described in detail below can be used as the steady-state breathing profile that is used in the process of determining a metabolic effect of the event on the subject.

According to examples of the presently disclosed subject matter, as part of determining the metabolic effect of the current event on the subject, the metabolic state of the subject while under the effect of the current event can be measured. According to examples of the presently disclosed subject matter, determining the metabolic state of the subject, under the effect of the current event, can include obtaining data relating to oxygen consumption and/or carbon dioxide production during the inhale-exhale cycle that met the correspondence criterion to determine a current metabolic state of the subject.

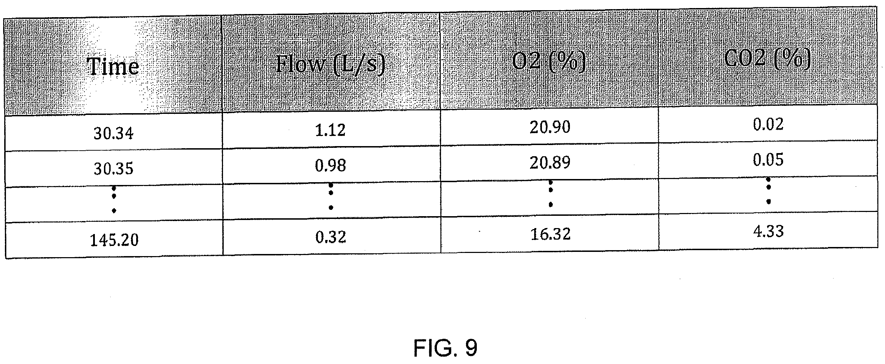

Reference is now made to **FIG. 9****,** which indicate a set of stored gas exchange measurements that were obtained as part of the method of determining a metabolic effect of an event on a subject. Each row represents a different measurement. For each measurement there is stored a timestamp which indicates the time when the set of measurements were taken the measured flow, given here in liters per second units, the concentration of oxygen and the concentration of carbon dioxide. It would be appreciated that are measurements can also be obtained and stored in accordance with examples of the presently disclosed subject matter. It would also be appreciated that similar data can be obtained and can be stored in a similar manner, or in a different manner for the method of determining a metabolic property which was described above.

According to examples of the presently disclosed subject matter, using the method of determining a metabolic property in a subject, which was described above, can enable the measurement of the metabolic state of the subject under the effect of the current event, since this method may be used in close time proximity to the occurrence of the event, and so the measurements taken by this method can provide a reliable indication of the event's effect of a metabolic property of the subject.

According to examples of the presently disclosed subject matter, in addition to determining the metabolic state of the subject while under the effect of the current event, reference metabolic data which is related to a reference metabolic state of the subject can be obtained. According to examples of the presently disclosed subject matter, the reference metabolic data can relate to the metabolic state of the subject during a reference metabolic state determination session that was performed while the subject's breathing was in a steady state, for example. In another example of the presently disclosed subject matter, the reference metabolic state data can represent a previous measurement of the metabolic state of the subject, while the subject was under the influence of an event of the same type which is currently affecting the subject.

Still further by way of example, the metabolic effect of the event on the subject is derived from a relation between the current metabolic state data and the reference metabolic state data.

In yet another example, the metabolic effect of the event on the subject can be determined by comparing the oxygen consumption and/or carbon dioxide production during the inhale-exhale cycle(s) that meets the correspondence criterion with the oxygen consumption and/or carbon dioxide production of the subject when the subject's breathing is in a steady state. For example, when determining the steady state breathing profile of a subject, the oxygen consumption and/or carbon dioxide production at the steady state can be determined and recorded. The oxygen consumption and carbon dioxide production values can be processed, e.g., compared, and the metabolic effect of the event can be determined based on the difference or based on some other relation between the current oxygen consumption and/or carbon dioxide production value and the oxygen consumption and/or carbon dioxide production value when the subject was in a steady breathing state.

In yet another example, the metabolic effect of the event on the subject can be determined by comparing the oxygen consumption and/or carbon dioxide production during the inhale-exhale cycle(s) that meets the correspondence criterion with an historic oxygen consumption and/or carbon dioxide production of the subject during a previous measurement. The oxygen consumption and/or carbon dioxide production values can then be processed, e.g., compared, and the metabolic effect of the event can be determined based on the difference or based on some other relation between the current and historic oxygen consumption and/or carbon dioxide production values. The historic oxygen consumption and/or carbon dioxide production value can be associated with the same event as the event under which effect the current oxygen consumption and carbon dioxide production measurement is taken, or it can be a different event, related or not to the current effect.

In yet another example, the metabolic effect of the event on the subject can be determined by comparing the oxygen consumption and/or carbon dioxide production during the inhale-exhale cycle(s) that meets the correspondence criterion with oxygen consumption and/or carbon dioxide production values taken from other subjects which were affected by the same event or by a different event, related to the event which the subject is influenced by or not related to it.

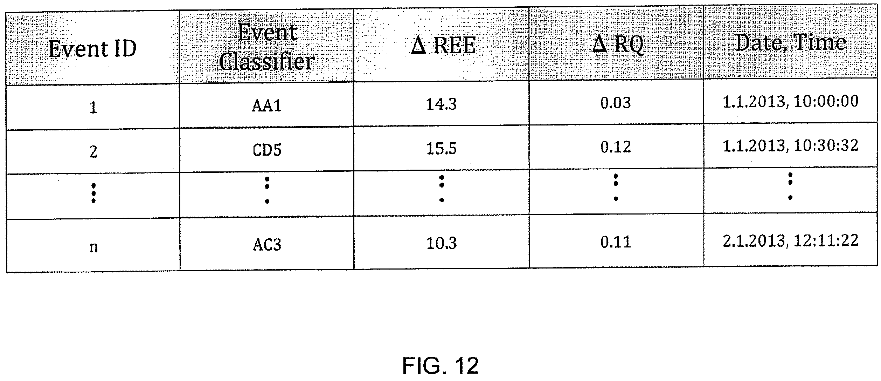

Reference is now made to **FIG. 12****,** which is a graphical illustration of a data structure in which various data related to recorded events can be kept, as part of some examples of the presently disclosed subject matter. According to examples of the presently disclosed subject matter, each record in the event records table can include a unique event ID (the event ID can serve as a primary key and it is assigned whenever new event data is received). Each record can also include an event classification code, which classifies the type of event to which the recorded event relates. As mentioned above, every type of event can be associated with a unique code. The event classification code can be used to access further data related to the various types of events, including for example, a description of the event type. Each event record can also include data relating to the metabolic effect of the event, which can be computed using the techniques described herein. In the repository shown in FIG. 12, the metabolic effect is ΔREE. By way of example, the metabolic effect data can be stored in a different repository, and the event record can include a link or a pointer to the metabolic effect data. In addition the date and time when the measurement was taken or obtained can be logged.

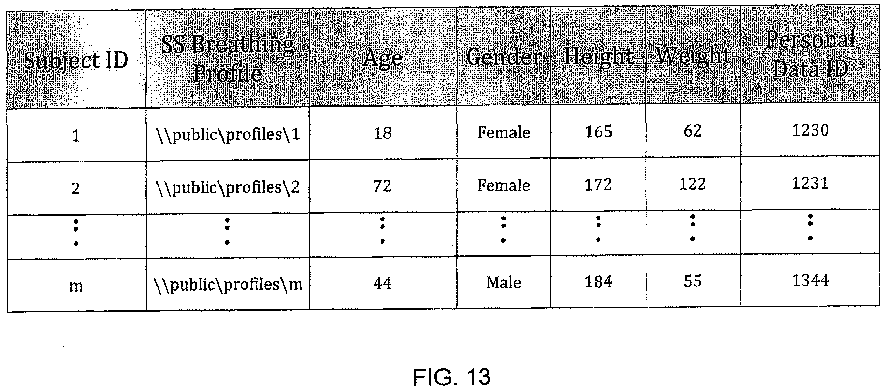

Referring now to **FIG. 13****,** which is a graphical illustration of a data structure in which various data related to different subjects can be kept, as part of examples of the presently disclosed subject matter. According to examples of the presently disclosed subject matter, the method of determining an effect of an event over metabolic properties of a subject can be implemented as a web-based service, and can store various data relating to effect of an event over metabolic properties for a plurality of different subjects. The various subjects can be capable of exchanging data with the web-based service through any appropriate digital communication device, such as a Smart Phone, a desktop computer, a laptop computer or even dedicated computer hardware.

According to examples of the presently disclosed subject matter, for each one of the plurality of subjects for which there is a record in the subjects data structure, the representative breathing profile of the subject can be kept. In some examples of the presently disclosed subject matter, for each one of the plurality of subjects for which there is a record in the subjects data structure a pointer or a link to a location where the representative breathing profile is stored can be maintained. The representative breathing profile can be used in the process of determining the effect of an event over metabolic properties of a subject, as described above.

According to examples of the presently disclosed subject matter, in addition to the subject ID and the representative breathing profile, the subjects data structure can hold further data, various personal details of the subject, such as age, gender, weight, height, medical history, etc., and possibly also a personal data ID, which can be used, for example, to access an entry in a separate table that is used to hold additional personal data of the user, including a table on an external node or platform, and including a table that is owned by a third party.

It will also be understood that the apparatus according to the invention may be a suitably programmed computer. Likewise, the invention contemplates a computer program being readable by a computer for executing the method of the invention. The invention further contemplates a machine-readable memory tangibly embodying a program of instructions executable by the machine for executing the method of the invention.

## Claims

1. A method, comprising:
obtaining data related to a current event;
obtaining a representative inhale-exhale cycle breathing volume over time data, where during which representative cycle, a subject's gas exchange represents a metabolic state of the subject;
obtaining a correspondence criterion related to the representative cycle;
while the subject is under influence of the current event, monitoring a subject's inhale-exhale cycle breathing volume over time over one or more inhale-exhale cycles;
when the subject performs at least one inhale-exhale cycle that meets the correspondence criterion related to the representative cycle, using data relating to oxygen consumption and/or carbon dioxide production during the inhale-exhale cycle that met the correspondence criterion to determine a metabolic effect of the event on the subject.

2. The method according to claim 1, further comprising:
obtaining reference metabolic data related to a first metabolic state of the subject;
using data relating to oxygen consumption and/or carbon dioxide production during the inhale-exhale cycle that met the correspondence criterion to determine a second metabolic state of the subject, and
computing the metabolic effect of the event on the subject from a relation between the first metabolic state of the subject and the second metabolic state of the subject.

3. The method according to claim 1, further comprising:
obtaining reference metabolic data which is related to a reference metabolic state of the subject, the reference metabolic data relates to a metabolic state of the subject during a reference metabolic state determination session that was performed while the subject's breathing was in a steady state and includes data relating to oxygen consumption and/or carbon dioxide production of the subject when the subject's breathing is in a steady state; and
and wherein said computing the metabolic effect of the event comprises processing data relating to the oxygen consumption and/or carbon dioxide production during the inhale-exhale cycle(s) that met the correspondence criterion with the reference metabolic data.

4. The method according to claim 3, wherein computing the metabolic effect of the event is determined based on a relation between the current oxygen consumption and/or carbon dioxide production value and the oxygen consumption and/or carbon dioxide production value when the subject was in a steady breathing state.

5. The method according to claim 3, wherein the reference metabolic state data represents a previous measurement of the metabolic state of the subject, while the subject was under the influence of an event of the same type which is currently affecting the subject.

6. The method according to claim 1, wherein computing the metabolic effect of the event is determined based on a relation between the current oxygen consumption and/or carbon dioxide production value and historic oxygen consumption and/or carbon dioxide production values.

7. The method according to claim 1, further comprising:
obtaining a steady state criterion;
measuring the subject's breathing volume over time during a first plurality of inhale-exhale cycles; and
processing data relating to the subject's breathing volume over time during the first plurality of inhale-exhale cycles to detect when two or more inhale-exhale cycles from said first plurality inhale-exhale cycles meet the steady-state criterion;
determining the subject's representative inhale-exhale cycle breathing volume over time data by processing breathing volume over time over the two or more inhale-exhale cycles that met the steady-state criterion.

8. The method according to claim 7, wherein processing breathing volume over time during the two or more inhale-exhale cycles that met the steady-state criterion, comprises:
computing a subject's representative inhale-exhale cycle breathing volume over time data based on the subject's breathing volume over time during the two or more inhale-exhale cycles that met the steady-state criterion;
computing an allowed deviation of breathing volume over time; and
computing a target inhale-exhale cycle breathing volume over time data based at least on the representative breathing profile and on the allowed deviation.

9. An apparatus for determining a metabolic effect of an event on a subject, comprising:
a storage module configured for storing data related to a current event;
the storage module is configured for storing data relative to a representative inhale-exhale cycle breathing volume over time data, where during which representative cycle, a subject's gas exchange represents a metabolic state of the subject;
the storage module is further configured to store a correspondence criterion related to the representative cycle;
a processing unit configured to (i) determine when the subject performs at least one inhale-exhale cycle that meets the correspondence criterion related to the representative breathing data, (ii) obtain data relating to oxygen consumption or carbon dioxide production during the inhale-exhale cycle that met the correspondence criterion, and (iii) determine, based on the data relating to oxygen consumption and/or carbon dioxide production and based on the stored data related to the current event, a metabolic effect of the event on the subject.

10. The apparatus according to claim 9, further comprising an input interface is configured to enable the subject to specify an event that the subject is currently under influence thereof.

11. The apparatus according to claim 9, wherein the storage module is configured to store reference metabolic data related to a first metabolic state of the subject, and wherein the processing unit is configured to compute the metabolic effect of the event on the subject from a relation between the first metabolic state of the subject and the second metabolic state of the subject.

12. The apparatus according to claim 9, wherein the storage module is configured to store reference metabolic data which is related to a reference metabolic state of the subject, the reference metabolic data relates to a metabolic state of the subject during a reference metabolic state determination session that was performed while the subject's breathing was in a steady state and includes data relating to oxygen consumption and/or carbon dioxide production of the subject when the subject's breathing is in a steady state, and wherein the processing unit is configured to process data relating to the oxygen consumption and/or carbon dioxide production during the inhale-exhale cycle(s) that met the correspondence criterion with the reference metabolic data.

13. The apparatus according to claim 12, wherein the reference metabolic state data represents a previous measurement of the metabolic state of the subject, while the subject was under the influence of an event of the same type which is currently affecting the subject.

14. The apparatus according to claim 9, wherein the processing unit is configured to determine the metabolic effect of the event based on a relation between the current oxygen consumption and/or carbon dioxide production value and historic oxygen consumption and/or carbon dioxide production values.

15. The apparatus according to claim 12, wherein the storage unit is further configured to store a steady state criterion, and wherein the processor is configured to measure the subject's breathing volume over time during a first plurality of inhale-exhale cycles, process data relating to the subject's breathing volume over time during the first plurality of inhale-exhale cycles to detect when two or more inhale-exhale cycles from said first plurality inhale-exhale cycles meet the steady-state criterion, and determine the subject's representative inhale-exhale cycle breathing volume over time data by processing breathing volume over time over the two or more inhale-exhale cycles that met the steady-state criterion.
